# EUROPEAN PATENT APPLICATION

(11) **EP 4 834 328 A1**
(43) Date of publication of application: **04.11.2026**
(21) Application number: 25173008.1
(22) Date of filing: 28.04.2025
(51) Int. Cl.: A61K 9/14, A61K 9/48, A61K 31/167, A61K 31/335, A61K 45/06, A61K 9/50

(54) **COMPOSITION**

(71) Applicant: Haleon CH SARL, 1197 Prangins (CH)
(72) Inventor: The designation of the inventor has not yet been filed
(74) Representative: Haleon Patent Department

(57) **Abstract**

The present invention relates to pharmaceutical compositions comprising paracetamol and cineole, particularly liquid pharmaceutical compositions. Particularly the compositions are provided in various formats, such as soft gels, hard-shell capsules, tablets, syrups, liquid suspensions and solutions, powder and granules to cater to different patient preferences and needs.

## Description

### FIELD OF THE INVENTION

The present invention relates to pharmaceutical compositions comprising paracetamol and cineole. Particularly the compositions are provided in various formats, such as capsules and soft gels, tablets, syrups, liquid formats, such as emulsions and suspensions, powder and granule formats, particularly for the preparation of oral solutions, thereby catering to different patient preferences and needs.

### BACKGROUND TO THE INVENTION

Paracetamol, also known as acetaminophen, is a widely used anti-pyretic sold in over-the-counter medication. In the United States, the Food and Drug Administration has stated that it is safe to consume up to a maximum dose of 4000 mg within 24 hours whilst European countries generally recommend no more than 3000 mg within 24 hours. This is because paracetamol is known to cause liver toxicity once above therapeutic levels in the blood.

However, given its ease of access in over-the-counter cold and flu medicines and prescription pain relievers, it is becoming more challenging for consumers to keep track of how much paracetamol they might have taken. In addition, many of these products are combination products, for example, containing pseudoephedrine or phenylephrine (both used as orally administered decongestants) for multi-symptom cold and flu relief. However, both pseudoephedrine and phenylephrine are under review by external authorities - pseudoephedrine for its role in the illicit production of methamphetamine and phenylephrine due to efficacy concerns when taken orally.

Thus, there remains a need for improved over-the-counter cold and flu formulations of paracetamol for oral administration that overcome the above challenges.

### SUMMARY OF THE INVENTION

In a First Aspect of the Invention, there is provided a pharmaceutical composition for oral administration comprising paracetamol (APAP), 1,8-cineole and one or more pharmaceutically acceptable excipients.

Particularly, the pharmaceutical composition comprises from 100mg to 1000mg of paracetamol and from 10mg to 400mg of 1,8-cineole per unit dose. In some embodiments, each single dose of paracetamol and 1,8-cineole is contained in one unit dosage form, for example as a powder for dissolution in water. In some embodiments, each single dose is contained in two unit dosage forms, for example in two soft-gel capsules or administered as two tablets.

In some embodiments, the 1,8-cineole is microencapsulated. More particularly, the microencapsulated 1,8-cineole comprises a gastro-resistant coating. Yet more particularly, the microencapsulated 1,8-cineole comprises a heat-resistant coating. Still yet more particularly, the microencapsulated 1,8-cineole comprises a coating that is both gastro-resistant and heat-resistant. Preferably, the heat-resistant coating is resistant to a temperature of up to 100°C.

Pharmaceutical dosage forms of the present Invention comprise one or more pharmaceutically acceptable excipients are selected from the group consisting of solvents, gelling agents, plasticisers, sweeteners, buffering agents, diluents, flavouring agents, preservatives, fillers, desiccants, stabilisers, emulsifiers, wetting agents, waxes and combinations thereof.

In some embodiments the pharmaceutical composition is provided in the form of a powder or granules for dissolution or reconstitution. Particularly, the powder for granules for dissolution or reconstitution are dissolved in or reconstituted with water, particularly warm water, particularly warm water having a temperature of from about 25°C, more particularly hot water having a temperature of from about 40°C up to about 100°C. Thus, the pharmaceutical composition provided in the form of a powder or granules for dissolution or reconstitution may be dissolved or reconstituted in water, particularly hot water, more particularly hot water having a temperature of from about 40°C up to about 100°C.

In other embodiments, the pharmaceutical composition is a liquid formulation, for example an aqueous pharmaceutical composition. In certain embodiments, the liquid formulation is a syrup. In certain embodiments, the liquid formulation is a soft gel, more specifically, a soft gel wherein the liquid formulation is hermetically sealed in a soft gelatin outer shell.

In a Second Aspect of the Invention, there is provided a pharmaceutical composition of the First Aspect for use in a method of treating or reducing pain and/or fever in a subject, comprising administering to the subject an effective amount of the pharmaceutical composition.

In some embodiments the pharmaceutical composition is for use in a method of treating or reducing multi-symptom pain in a subject. In some embodiments, the pharmaceutical composition is for use in a method of treating or reducing multi-symptom pain in a subject, wherein the multi-symptom pain is associated with respiratory infections particularly selected from the group consisting of common cold, and flu. In some embodiments, the pain is headache and/or muscle pain associated with respiratory infections such as common cold and flu.

In some embodiments the pharmaceutical composition is for use in a method of treating pain and/or fever in a subject, wherein the pain and/or fever is associated with respiratory infection, for example associated with respiratory infection selected from the group consisting of common cold and flu.

In some embodiments the pharmaceutical composition is for use in a method of treating pain and/or nasal congestion and/or increased mucous production in a subject, wherein the pain and/or nasal congestion and/or increased mucous production is associated with respiratory infection, particularly selected from the group consisting of common cold and flu.

In some embodiments the pharmaceutical composition is for use in a method of providing symptomatic relief of cold or flu symptoms such as cough, comprising administering to a patient in need thereof a therapeutically effective amount of the composition.

In some embodiments the pharmaceutical composition is for use in a method of providing symptomatic relief of cold or flu symptoms such as increased mucous production, comprising administering to a patient in need thereof a therapeutically effective amount of the composition.

In some embodiments, the pharmaceutical composition is for use in treating or reducing headache pain in a subject. In some embodiments, the pharmaceutical composition is for use in treating or reducing sinus pain in a subject. In some embodiments, the pharmaceutical composition is for use in treating or reducing pain arising from nasal congestion arising from cold or flu in a subject. In some embodiments, the pharmaceutical composition is for use in treating or reducing pain arising from increased mucous production arising from cold or flu in a subject.

In some embodiments, the pharmaceutical composition is for use in fever reduction and/or symptomatic relief of common cold and flu.

In a Third Aspect of the Invention, there is provided a pharmaceutical composition of the First Aspect for use in a method of reducing the risk of paracetamol induced liver damage in a subject.

### DETAILED DESCRIPTION OF THE INVENTION

The present invention relates to compositions comprising paracetamol (APAP) and cineole, specifically 1,8-cineole.

Cineole has a number of therapeutic effects such that the Inventors have discovered that it may be used as an alternative to pseudoephedrine or phenylephrine in over-the-counter cold and flu medications. Thus, a combination of paracetamol and cineole, particularly in a liquid formulation, aims to leverage the analgesic and antipyretic properties of paracetamol with the multifaceted therapeutic benefits of cineole. Without wishing to be bound by theory, the present inventors further believe that cineole may have a protective effect, potentially reducing the likelihood of paracetamol induced liver damage, for example if too much paracetamol is inadvertently ingested.

### Paracetamol

Paracetamol, a well-known drug used to treat pain and fever, is also known as N-acetyl-p-aminophenol, acetaminophen and APAP. Paracetamol has the following structure:

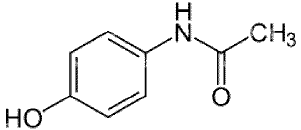

### Cineole

Cineole, also known as 1,8-cineole, is a naturally occurring monoterpene found in the essential oils of various plants, including eucalyptus, rosemary, and camphor laurel. Cineole is known for its diverse therapeutic properties, including anti-inflammatory, antioxidant and bronchodilatory action. Particularly, 1,8-cineole has the following structure:

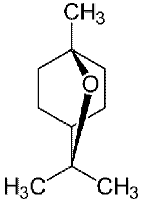

The term "oral administration" refers to the provision of the pharmaceutical composition via the mouth through ingestion Thus, the dosage forms are for oral administration, via the mouth, intended to be delivered to or released in the stomach and/or small intestine of a patient.

Particularly, the pharmaceutical compositions of the Invention comprise from 100mg to 1000mg of paracetamol and from 10mg to 400mg of 1,8-cineole.

The pharmaceutical compositions may comprise from 100mg to 1000mg of paracetamol, such as 100mg, 150mg, 200mg, 250mg, 300mg, 325mg 350mg, 400mg, 500mg, 550mg, 600mg, 650mg, 700mg, 750mg, 800mg, 850mg, 900mg, 950mg or 1000mg. In certain preferred embodiments, the composition comprises 500mg paracetamol.

The pharmaceutical compositions may comprise from 10mg to 400mg of 1,8-cineole, such as 10mg, 15mg, 20mg, 25mg, 30mg, 35mg, 40mg, 45mg, 50mg, 55mg, 60mg, 65mg, 70mg, 75mg, 80mg, 85mg, 90mg, 95mg, 100mg, 125mg, 150mg, 175mg, 200mg, 225mg, 250mg, 275mg, 300mg, 325mg, 350mg, 375mg or 400mg. In certain preferred embodiments, the composition comprises 200mg of 1,8-cineole.

Particularly, the pharmaceutical compositions of the Invention comprise from 250mg to 1000mg of paracetamol and from 100mg to 400mg of 1,8-cineole. More particularly the compositions of the Invention may comprise 500mg paracetamol and 200mg of 1,8-cineole.

Cineole can have a strong flavour and smell that some consumers dislike, often having a "repeating" taste or burps, limiting the use of this compound in oral over-the-counter medications. The Inventors have discovered that microencapsulating cineole can overcome these issues, enabling cineole release at an enteric level. Surprisingly, the use of microencapsulation also enables the provision of cineole in a number of pharmaceutical formats, particularly liquid formats, including soft-gels, syrups, and drinks, such as hot drinks. Advantageously, microencapsulating cineole further allows these product formats to be flavoured according to consumer preferences.

Microencapsulation refers to physicochemical processes to entrap an active compound in a material in order to improve its functionality. In some embodiments, microencapsulation is performed by coating a liquid with a protective wall material that separates the liquid from the external environment. The liquid contained within the wall material is known as the core, and the complete microencapsulated particle is known as a microcapsule. Microencapsulation is a technique known in the art. Physical processes for microencapsulation may use mechanical means to entrap the liquid core within the wall materials. By way of non-limiting example, typical physical methods include spray drying, air suspension coating, spray cooling and chilling, co-crystallization, and centrifugal extrusion. Chemical methods for encapsulation are also known.

Thus, particularly, the 1,8-cineole is microencapsulated. More particularly, the microencapsulated 1,8-cineole comprises a gastro-resistant coating. Yet more particularly, the microencapsulated 1,8-cineole comprises a heat-resistant coating. Still yet more particularly, the microencapsulated 1,8-cineole comprises a coating that is both gastro-resistant and heat-resistant. Preferably, the heat-resistant coating is resistant to a temperature of up to 100°C. Microcapsules of the present Invention comprise a core material of 1,8-cineole surrounded by a shell. Particularly microcapsules of 1,8-cineole have a mean particle size of from 0.001µm to 1000µm.

In some embodiments 1,8-cineole is encapsulated using gelatin, cellulose, high melt fats or waxes.

The pharmaceutical dosage form may be a capsule, such as a hard-shell capsule or soft-gel capsule, tablet, syrup or pharmaceutical beverage product, particularly a powder or granule formulation for dissolution or reconstitution with hot water.

The term "soft gel" generally refers to a one-piece, hermetically sealed soft outer shell containing a filling composition. In some embodiments the soft-gel is a one-piece, hermetically sealed soft gelatin outer shell containing a filling composition.

The term "hard-shell capsule" refers to a capsule having telescopically engaging body and cap portions, for example made of gelatin optionally with a plasticiser such as glycerin and/or sorbitol.

In certain embodiments, the pharmaceutical dosage form may be a capsule in capsule format, particularly comprising a first capsule comprising paracetamol and being soluble in or disintegrated by the acidic secretions of the stomach and a second capsule within the first capsule, comprising cineole and being soluble in or disintegrated by the alkaline intestinal secretions but being substantially insoluble or resistant to the acid secretions of the stomach.

The term "syrup" means a pharmaceutical formulation having a viscosity such that it can flow without applied pressures but is sticky or tacky to the touch.

The term "powder or granules for dissolution or reconstitution" according to the present Invention refers to a powder or granules used for the preparation of a stable solution or suspension by adding a suitable volume of water, for example warm water, particularly hot water. Particularly, the powder or granules for dissolution or reconstitution is dissolved in or reconstituted with potable (drinking) water, particularly hot water, more particularly hot water having a temperature of from about 40°C up to about 100°C. Thus, the pharmaceutical composition provided in the form of a powder for reconstitution may be dissolved in water, particularly hot water, more particularly hot water having a temperature of from about 40°C up to about 100°C.

Pharmaceutical dosage forms of the present Invention comprise one or more pharmaceutically acceptable excipients. Pharmaceutically acceptable excipients may be solvents, gelling agents, plasticisers, sweeteners, buffering agents, diluents, flavouring agents, preservatives, fillers, desiccants, waxes, stabilisers, emulsifiers, wetting agents and the like. By way of non-limiting example, suitable pharmaceutically acceptable excipients include, purified water, polyethylene glycol, gelatin, glycerol, sorbitol, propylene glycol, silicon dioxide, triglycerides, titanium dioxide, polymers, polysorbate and the like. Thus, pharmaceutical dosage forms of the present Invention may comprise one or more pharmaceutically acceptable excipients selected from the group consisting of solvents, gelling agents, plasticisers, sweeteners, buffering agents, diluents, flavouring agents, preservatives, fillers, desiccants, waxes, stabilisers, emulsifiers, wetting agents and combinations thereof.

For the avoidance of doubt, reference to "% by weight" (%w/w) means by weight of the total composition, the amount of each ingredient being selected so that total ingredients in the composition sum to 100 percent by weight.

### General

The term "comprising" encompasses "including" e.g. a composition "comprising" X may include something additional e.g. X + Y. In some implementations, the term "comprising" refers to the inclusion of the indicated active agent, such as recited compounds, as well as inclusion of other active agents, and carriers, excipients, emollients, stabilizers, etc., known in the consumer health industry and generally recognized as safe (GRAS). Use of the transitional phrase "consisting essentially of" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. All percentages and ratios used herein are by weight of total composition, unless otherwise indicated.

All references or patent applications cited within this patent specification are incorporated by reference herein. In order that this invention may be better understood, the following examples are set forth. These examples are for purposes of illustration only and are not to be construed as limiting the scope of the invention in any manner.

### EXAMPLES

### Example 1: Soft Gel

| **INGREDIENT** | **QUANTITY mg/capsule** | **FUNCTION** | **REFERENCE TO STANDARD** |
|---|---|---|---|
| Paracetamol | 500 | Active substance | Ph.Eur. |
| 1,8 cineole microencapsulated* | 200 | Active substance | ASMF |
| Polyethylene Glycol | 390 | Solvent | Ph Eur |
| Purified water | 60 | Solvent | USP/NF, PhEur |

| **Capsule Shell Mass** | | | |
|---|---|---|---|
| Gelatin | 128 | Gelling agent | USP/NF, PhEur |
| Glycerol | 31 | Plasticizer | Ph.Eur |
| Sorbitol-solution | 31 | Plasticizer | USP/NF, PhEur |
| Purified water | 110 | Solvent | USP/NF, PhEur |

A liquid formulation is prepared by stirring polyethylene glycol at high speed (e.g., 2500-3500 revolutions per minute) and slowly heating to 75^{O}C after which purified water is added followed by paracetamol. The mixture is stirred for 30 minutes until it becomes clear. The clear solution is then removed from the heat source, 1,8-cineole microencapsulated is added with stirring and the liquid formulation de-aerated in a vacuum desiccator (a vacuum between 26 to 30 inches of mercury) at room temperature for 20-30 minutes.

The liquid formulation is encapsulated in soft gelatin shells to form soft-gel capsules using a conventional rotary die process. Suitable soft gelatin shells may comprise (i) gelatin, 35-60 percent by weight; (ii) glycerol, 10-15 percent by weight; (iii) sorbitol, 11-20 percent by weight; (iv) purified water, 20- 40 percent by weight.

The soft-gel capsules of the invention can also be prepared by other methods well known in the art.

### Example 2: Soft Gel II

| **INGREDIENT** | **QUANTITY mg/capsule** | **FUNCTION** | **REFERENCE TO STANDARD** |
|---|---|---|---|
| Paracetamol | 500 | Active substance | Ph Eur |
| Polyethylene Glycol 400 | 418 | Solvent | Ph Eur |
| Propylene Glycol | 20 | Solvent | Ph Eur |
| Colloidal Silicon Dioxide | 2 | Processing Aid | Ph Eur |
| 1,8 cineole | 200 | Active substance | ASMF |
| Triglycerides, medium chain | 100 | Solvent | Ph.Eur |
| Gelatin | 252 | Gelling agent | USP/NF, PhEur |
| Gelatin | 100 | | |
| Glycerol 85% | 23.333 | Plasticizer | Ph.Eur |
| Sorbitol-solution | 108 | Plasticizer | USP/NF, PhEur |
| Sorbitol-solution | 23.333 | | |
| Purified water | 10 + 72.222 | Solvent | USP/NF, PhEur |
| Titanium Dioxide | 12 | | |

| **Coating I** | | | |
|---|---|---|---|
| Methacrylic acid - ethyl acrylate copolymer (1:1) dispersion 30% (Eudragit L30 D55) | 69.701 | Coating dispersion | Ph.Eur |

| **Coating II** | | | |
|---|---|---|---|
| Triethyl citrate | 4.182 | plasticizer | Ph.Eur |
| Glycerol monostearate | 1.568 | Glidant | Ph.Eur |
| Polysorbate 80 | 0,418 | Emulsifier | Ph.Eur |
| Purified water | 59.522 | Solvent | Ph.Eur |

To prepare the liquid formulation, PEG-400 and propylene glycol are mixed with stirring at high speed (e.g., from 250-to 4500 revolutions per minute). The mixture is slowly heated to 75^{O}C and the colloidal silicon dioxide is then added to the mixture at the same temperature. After the mixture turns into a clear solution with constant stirring, the purified water and triglycerides are added, followed by addition of paracetamol. The solution is stirred a until it becomes clear, removed from the heat and the 1,8-cinole is added with stirring. The solution is de-aerated in a vacuum desiccator (a vacuum between 26 to 30 inches of mercury) at room temperature for 20-30 minutes.

The liquid formulation can be encapsulated in soft gelatin shells to form soft-gel capsules using a conventional rotary die process. The filled soft-gel capsules are finished by coating with coating I and II.

The softgel capsules of the invention can also be prepared by other methods well known in the art.

### Example 3: Powder Formulation for dissolution

| **INGREDIENT** | **QUANTITY mg/unit** | **FUNCTION** | **REFERENCE TO STANDARD** |
|---|---|---|---|
| Paracetamol | 500 | Active substance | Ph Eur |
| 1,8 cineole microencapsulated* | 200 | Active substance | ASMF |

| Other ingredients | | | |
|---|---|---|---|
| Sucrose | 1020 | Sweetener | Ph.Eur. |
| Citric acid | 685 | Buffering agent | Ph.Eur. |
| Maltodextrin | 200 | Diluent | Ph.Eur. |
| Flavour | 200 | Flavour | HSE (in house standard) |
| Sodium citrate | 140 | Buffering agent | Ph.Eur. |
| Acesulfame K (Potassium) | 35 | Sweetener | Ph.Eur. |
| Silicon Dioxide (Silica, Colloidal) | 20 | Desiccant /Moisture Scavenger | Ph.Eur. |

All raw materials are passed through appropriately sized screens to ensure the product is free from large agglomerates. The screened raw materials are then blended in an appropriate size blender for an appropriate blending time to ensure proper blend uniformity. After blending, the powder blend is filled into sachets using conventional sachet-filling equipment. The sachets are then heat-sealed and placed in secondary packaging. Before use, the powder is dissolved in water.

The purpose of the above description is to illustrate some embodiments of the present invention without implying a limitation. It will be apparent to those skilled in the art that various modifications and variations may be made in the apparatus or procedure of the invention without departing from the scope or spirit of the invention.

## Claims

1. A pharmaceutical composition for oral administration comprising paracetamol (APAP), 1,8-cineole and one or more pharmaceutically acceptable excipients.

2. The pharmaceutical composition of claim 1 comprising from 100mg to 1000mg of paracetamol and from 10mg to 400mg of 1,8-cineole, particularly about 500mg of paracetamol and about 200mg of 1,8-cineole.

3. The pharmaceutical composition of claim 1 or 2, wherein the 1,8-cineole is microencapsulated.

4. The pharmaceutical composition of claim 3, wherein the microencapsulated 1,8-cineole comprises a gastro-resistant and/or heat-resistant coating.

5. The pharmaceutical dosage form of any preceding claim, wherein the one or more pharmaceutically acceptable excipients are selected from the group consisting of solvents, gelling agents, plasticisers, sweeteners, buffering agents, diluents, flavouring agents, preservatives, fillers, desiccants, waxes, stabilisers, emulsifiers, wetting agents and combinations thereof.

6. The pharmaceutical composition of Claim 5 being a powder or granules for reconstitution.

7. The pharmaceutical composition of Claim 6 dissolved in water, particularly hot water.

8. The pharmaceutical composition of claim 5 being a liquid formulation, particularly an aqueous formulation.

9. The pharmaceutical composition of Claim 8 wherein the liquid formulation is a syrup.

10. The pharmaceutical composition of Claim 8, wherein the liquid formulation is a soft gel.

11. The pharmaceutical composition of claim 5 being a hard-shell capsule.

12. The pharmaceutical composition of claim 5 being a tablet.

13. The pharmaceutical composition of any one of claims 1 to 12 for use in a method of treating pain and/or fever in a subject, comprising administering to the subject an effective amount of the pharmaceutical composition.

14. The pharmaceutical composition of claim 13 for use in a method of treating pain in a subject wherein the pain is selected from the group consisting of headache and/or muscle pain associated with common cold or flu.

15. The pharmaceutical composition of claim 13 for use in a method of treating pain and/ or fever in a subject, wherein the fever is associated with respiratory infection, particularly wherein the respiratory infection is selected from the group consisting of common cold and flu.

16. The pharmaceutical composition of any one of claims 1 to 12 for use in a method of providing symptomatic relief of respiratory infection such as cough, comprising administering to a patient in need thereof a therapeutically effective amount of the composition.
